# EUROPEAN PATENT SPECIFICATION

(11) **EP 3 137 867 B1**
(45) Date of publication and mention of the grant of the patent: **22.04.2020**
(21) Application number: 15728207.0
(22) Date of filing: 01.05.2015
(51) Int. Cl.: A61B 5/0215, G01L 7/06, G01L 7/18, G01L 11/02, G01H 9/00, G01L 1/24

(54) **OPTICAL FIBER SENSOR ASSEMBLY**
GLASFASERSENSORANORDNUNG
ENSEMBLE CAPTEUR À FIBRE OPTIQUE

(30) Priority: 01.05.2014 NL 1040788
(43) Date of publication of application: 08.03.2017
(73) Proprietor: Optics11 B.V., 1081 HV Amsterdam (NL)
(72) Inventor: KARABACAK, Devrez Mehmet, NL-2264 SG Leidschendam (NL); VAN RIEL, Paul, NL-2264 SG Leidschendam (NL); KNOPPERS, German Enrique, NL-2264 SG Leidschendam (NL); MEULBLOK, Bastiaan, NL-2264 SG Leidschendam (NL); FREY, Steven R., NL-2264 SG Leidschendam (NL)
(74) Representative: V.O.
(86) International application number: PCT/NL2015/050302
(87) International publication number: WO 2015/167340

(56) References cited:
- EP-A1- 2 491 883
- WO-A1-02/082010
- WO-A1-2010/136724
- DE-U1-202014 100 938
- US-A1- 2002 154 860
- US-A1- 2002 196 993
- US-A1- 2009 324 161
- US-A1- 2011 087 112
- US-A1- 2011 178 413
- US-B1- 6 201 912

## Description

### FIELD OF THE INVENTION

The present invention relates in general to a method and device for sensing physical parameters, and a method for improving the sensitivity of an FBG sensor. In a particular embodiment, the invention relates to the field of sensing pressure and/or pressure variations in a fluid, typically a liquid or a gas.

### BACKGROUND OF THE INVENTION

It is a general desire to be able to measure various physical parameters, and to be able to do so there is a need for a sensor that is responsive to such physical parameter to be measured. The sensor should ideally be accurate and have a response time allowing stationary as well as dynamic values to be easily measured.

It is well known that an optical fiber provided with a Fiber Bragg Grating (FBG) is capable of providing a measuring signal that accurately follows fiber length variations at the location of the FBG. Length and length variations of the FBG can be determined from its peak frequency using a laser interrogator.

A typical application of FBGs is measuring pressure and pressure variations. However, known devices that comprise optical pressure sensors are relatively complicated and bulky. For instance, a known pressure sensor system comprises an optical fiber wound around a drum, that expands or contracts with pressure variations; such design necessarily has a size at least larger than the drum size.

By way of non-limiting example, steam-assisted oil extraction is a field of technology where it is desirable to be able to sense pressure and/or pressure variations. This technology typically involves long pipes (several hundreds of meters) arranged in the ground, in which high-pressure high-temperature steam is used to extract crude oil and/or bitumen from the surrounding ground and push it through a recovery pipe. Often, in the steam injection pipe, a detection channel is arranged, which typically has an inner diameter of about 8 mm, and which is available for arranging a sensing system therein. This means that the operating conditions for such sensing system include high pressure (typically 100 bar and more), high temperature (typically 320 °C and higher), aggressive atmosphere (steam and oil), and small transverse dimensions and large axial dimensions.

Sensing systems for such conditions have already been proposed. By way of example, reference is made to US patent application 20050195402, which describes a Fabry-Perot-based sensor device having an elongate narrow housing with a sensing chamber located at the tip, confined by a thin membrane that is responsive to pressure. Such design, however, suffers from disadvantages that it provides only one single measuring spot, is not very sensitive, and is not suitable for dynamic pressure variations in the acoustic frequency range.

It is noted that patent publication WO 2010/136724 A1 discloses a Bragg grating fiber hydrophone, including a fluid chamber and an optical fiber passing through said chamber. The fluid chamber is defined by a casing that is provided, at both axial ends, with extensible and compressible tubes extending along the longitudinal axis.

It is further noted that patent publication US 2002/0154860 A1 discloses a fiber grating pressure sensor including an optical fiber having a Bragg grating impressed therein which is encased within and fused to at least a portion of a glass capillary tube.

It is noted that patent publication US 2002/196993 A1 discloses a fiber optic supported sensor-telemetry system for oilfield monitoring applications, where an optical fiber provides telemetry of signals outputted by both optical sensors based on a fiber Bragg grating and non-optical sensors based on micro-electro-mechanical systems and micro-optoelectro-mechanical systems.

It is also noted that patent publication US 2011/0178413 A1 discloses a frequency-domain optical coherence tomography (FD-OCT) system, includes a first, second and third optical channel receiving an optical beam from a wavelength-swept laser 34.

It is noted that German patent publication DE 20 2014 100 938 U1 and American patent publication US 2011/0087112 A1 disclose a medical catheter including a Fiber Bragg Grating for measuring pressure in a patient's tissue.

### SUMMARY OF THE INVENTION

It is an objective of the present invention to apply the advantages of FBG-based optical sensors in a sensor design that can accurately measure various physical parameters.

It is a further objective of the present invention to provide a sensor design that is robust and small, and relatively easy to manufacture.

It is a further objective of the present invention to provide a method for improving the sensitivity of FBG-based optical sensors.

The present invention aims particularly to provide a pressure sensing system that is capable of sensing pressure, static as well as dynamic up to acoustic frequencies at high sensitivity, and that has a very narrow shape and sufficient flexibility to be entered into a narrow and curved channel.

According to the invention, a sensor assembly according to claim 1 is provided.

According to an important aspect of the present invention, a sensing body is provided, at least partly made from a material of which the size is sensitive to a physical parameter under investigation, i.e. this material contracts or expands with variations in this physical parameter. The sensing body is attached at two points of an optical fiber, at opposite sides of an FBG. Thus, the value of the physical parameter being measured is transformed to a certain length of the part of the fiber on which the FBG is etched. The sensing body can be made to have a very slim profile.

In a specially preferred embodiment, the optical fiber is arranged in a narrow capillary filled with glue, and the sensing body is attached to the capillary. The capillary serves to protect the fiber. In a particular embodiment, the capillary is omitted at the location of the FBG, so that length variations of the fiber are concentrated in the FBG to increase the measuring sensitivity.

### BRIEF DESCRIPTION OF THE DRAWINGS

These and other aspects, features and advantages of the present invention will be further explained by the following description of one or more preferred embodiments with reference to the drawings, in which same reference numerals indicate same or similar parts, and in which:
figure 1A schematically shows a longitudinal cross section of a first embodiment of a sensing assembly;
figure 1B is a section comparable to figure 1A showing a variation of the embodiment of figure 1A;
figure 2A schematically shows a longitudinal cross section of a second embodiment of a sensing assembly;
figure 2B is a section comparable to figure 2A showing a variation of the embodiment of figure 2A;
figure 2C is a section comparable to figure 2A showing a variation of the embodiment of figure 2A;
figure 3A schematically shows a longitudinal cross section of a third embodiment of a sensing assembly;
figure 3B is a section comparable to figure 3A showing a variation of the embodiment of figure 3A;
figure 4A schematically shows a longitudinal cross section of a fourth embodiment of a sensing assembly;
figure 4B is a section comparable to figure 4A showing a variation of the embodiment of figure 4A;
figure 5A schematically shows a longitudinal cross section of a fifth embodiment of a sensing assembly;
figure 5B is a section comparable to figure 5A showing a variation of the embodiment of figure 5A;
figure 6 schematically shows an oil well bore with a sensing system.

### DETAILED DESCRIPTION OF THE INVENTION

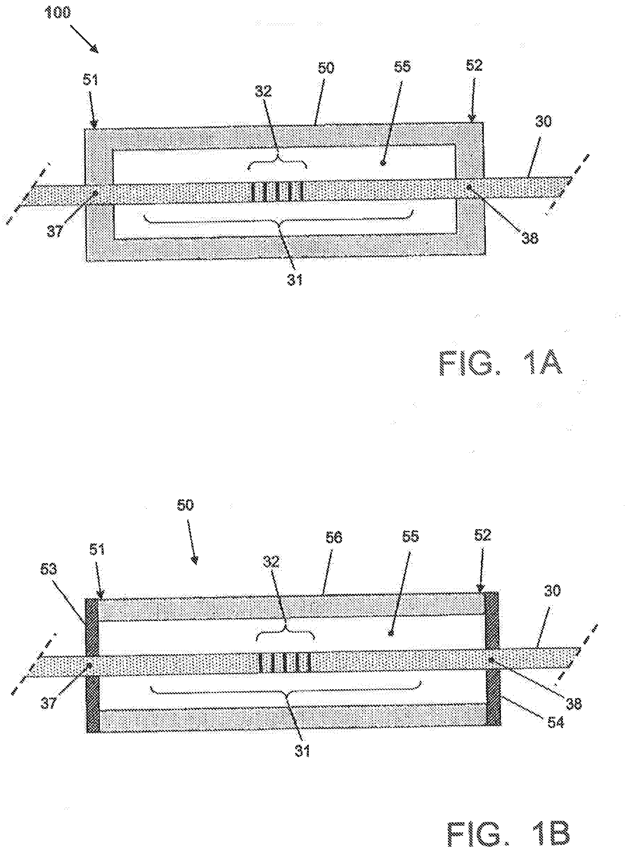

Figure 1A schematically shows a longitudinal cross section of a first embodiment of an elongate sensing assembly 100 according to the present invention. The sensing assembly 100 comprises an optical fiber 30 having a sensing fiber portion 31 that comprises at least one Fiber Bragg Grating 32 (FBG). The figure illustratively shows only one FBG, but the sensing fiber portion 31 may have two or more FBGs.

Optical fibers with FBGs are known per se, therefore an explanation thereof will be kept brief here. An FBG in essence consists of a series of material modifications arranged lengthwise in the fiber. Normally, the optical properties of an optical fiber are constant along the length, which optical properties include the refractive index. Such material modification, however, has a slightly different refractive index. A plurality of such material modifications, at mutually the same distance, behaves as a grating, which typically is reflective for a small wavelength band. If a light pulse is made to enter the fiber, substantially all wavelengths will pass the grating location but light within said small wavelength band will be reflected. At the input end of the fiber, a reflected light pulse will be received, of which the wavelength is indicative for the mutual distance between the successive material modifications, which will also be indicated as the grating pitch. The FBG can therefore be considered to be a narrowband reflector.

Such FBG reflector sensor is typically sensitive to (local) strain. Variations in strain cause variations in length of the fiber, including variations in grating pitch. These distance variations, in turn, translate to variations in the wavelength of the reflected light.

The strain condition of an FBG can thus be measured by providing an external light beam or pulse, using an external light source, making this light beam or pulse enter an input end of the fiber, and detecting any reflected light. Such procedure is indicated as "interrogation" of the FBG sensor by external light. The FBG response to this interrogation is reflecting light of which the wavelength matches the grating. It is also possible that the FBG sensor is used as mirror portion in a fiber laser, so that the laser output wavelength matches the grating; the laser may for instance be a distributed feed back (DFB) fiber laser, or a distributed Bragg reflector (DBR) fiber laser. The wavelength spectrum of a fiber laser looks similar, qualitatively, to the single wavelength reflection spectrum of a reflector. The present invention is applicable in either type of measurement procedure.

Since the present invention can be implemented using optical fibers with FBG reflectors of the same type as currently are being deployed, a more detailed explanation of design and manufacture of optical fibers with FBG reflectors is omitted here.

Apart from the sensing fiber portion 31 shown, the fiber 30 may have further sensing fiber portions at other positions, but this is not shown. In fact, the fiber 30 may have a plurality of such sensing fiber portions distributed over a large length portion. And, each sensing fiber portion may have one or more FBGs. Thus, the fiber 30 may be provided with multiple FBGs. If these FBGs have mutually different grating pitch, the reflected wavelengths will be mutually different, and it will be possible to perform multiple measurements at the same time.

For being able to sense a certain physical parameter, a further aspect of the sensing assembly 100 is a sensing body 50 attached to the fiber 30. More particularly, the sensing body 50 has body ends 51, 52 arranged at opposite sides of the sensing portion 31 and attached directly to the fiber 30; the positions of the fiber where the sensing body ends 51, 52 engage will be indicated as attachment points 37, 38. For instance, the sensing body 50 may have its ends 51, 52 clamped firmly to the fiber 30. Although it is for instance possible that the sensing body 50 is located adjacent the fiber 30, for sake of symmetry it is preferred that the sensing body 50 surrounds the fiber 30 and has a hollow inner space 55 in which the fiber 30 can move freely. Dependent on the application, the hollow inner space 55 can be air-filled or gas-filled or can be filled with a fluid or gel or in other ways.

It is to be noted that the material of the sensing body 50 should, at the operative temperature, be sufficiently rigid. It is further noted that the fiber 30 is held along a straight line between the attachment points 37, 38.

The sensing body 50 is made of a sensing material that is selected for being size-responsive to the physical property being measured by contracting or expanding in proportion to changes in the said physical property being measured. For example, to measure temperature the sensing body 50 can be made of a material that expands or contracts with a change of temperature. To measure pressure or acoustic waves, it can be made of a material that contracts and expands with increasing or decreasing pressure. Further examples are materials sensitive to change in chemical concentration and materials sensitive to changes in magnetic field strength.

Length variations of the sensing body 50 will be transformed to length variations of the fiber 30, which will be measurable by an interrogating light beam, as should be clear to a person skilled in the art.

In a preferred embodiment, the sensing body 50 is shaped as a cylinder, but this is not essential.

In a preferred embodiment, the inner space 55 of the sensing body 50 is shaped as a cylinder, but this is not essential. In such case, the diameter of the inner space 55 is larger than the diameter of the fiber 30.

In an embodiment, the sensing body 50 is a mono-body, i.e. manufactured as an integral body of single material, as illustrated in figure 1A. Figure 1B schematically illustrates a variation of this embodiment, where the sensing body 50 comprises a cylindrical portion 56 of which the opposite ends are bonded to terminating plates 53, 54 which consist of a material that is rigid relative to the sensing material of the cylindrical portion 56. Said plates in turn are bonded to the fiber 30. Such embodiment is particularly useful when the material of the sensing body 50 is relatively soft. Further, the embodiment of figure 1B is generally easier to manufacture

It is noted that in all cases a contraction or expansion of the sensing body 50 along the direction of the fiber 30 is translated to a change in length of the sensing fiber portion 31. The force exerted to the fiber by the expansion or contraction of the sensing body 50 material can be increased by thickening the sensing body 50. Hence the sensitivity of sensor assembly 100 will in most cases increase with thickening the sensing body 50.

In a preferred embodiment, the sensing material is shaped as a cylinder, as mentioned. In most cases this maximizes the transfer of contraction or expansion of the sensing material along the fiber to a change in length of the fiber. It is however not a requirement. For example the sensing material may be available in the form of rods. These can be mounted on the terminating plates 53, 54 to achieve the desired effect.

Also the terminating plates 53, 54 can be replaced by another termination of any shape as long as it is bonded to the fiber and transfers contraction or expansion of the sensing body to the fiber.

If the sensing material of sensing body 50 is sufficiently rigid, the terminating plates 53, 54 may be omitted and the sensing material of the sensing body 50 can be bonded to the fiber directly by various methods including welding and gluing. For instance, certain sensing material can be hardened for example by exposing it to heat, radiation, glue, chemicals, light or other external agent.

An important advantage of the design of the sensing assembly 100 is that it is a very small and flexible design, allowing it to be arranged in narrow and curved pathways. For instance, the outer diameter of the sensing body 50 may be as small as 1-5 mm. The design allows to apply a plurality of FBG-based sensors along a single length of fiber, and to arrange these fibers with multiple sensors in 1-, 2-, or 3- dimensional arrays of any shape. It is thus possible to measure a certain physical parameter at a plurality of positions and/or to measure a spatial distribution (scalar field or vector field).

Although it is within the scope of the present invention that all sensing bodies are made of the same sensing material, so that all sensing bodies are sensitive for the same physical parameter, it is also possible to apply sensing bodies made of mutually different sensing material such as to measure different physical properties in parallel.

In the above, it has been explained how variations in a physical parameter may cause length variations of the sensing body 50, and how the body 50 material may be selected to have appropriate sensitivity for a certain physical parameter. One particularly important physical parameter is the ambient pressure in a medium in which the sensing assembly 100 is disposed. The ambient pressure will exert compressive axial forces, i.e. forces directed along the fiber axis, on the axial end faces of the sensing body 50, of which the magnitude will depend on the diameter of the body 50, and variations in this pressure will result in a length variation response of the sensing body 50, with the amount of length variation depending on the combined stiffness of the fiber 30 and the body 50. This is because the sensing body 50 and the fiber 30 are mechanically connected in parallel with respect to the axial forces. For increasing the pressure sensitivity, in a further elaboration of the invention, it is proposed to reduce the axial stiffness of the sensing body 50 while maintaining the radial stiffness. As a result, the combined stiffness of the fiber 30 and the body 50 will be lower, so length variations in the fiber 30 caused by pressure acting on the axial end faces of the cylinder 50 will now effect a larger length variation in the fiber 30.

One feature of reducing the axial stiffness of the sensing body 50 is to have the wall thickness of the sensing body 50 as small as possible.

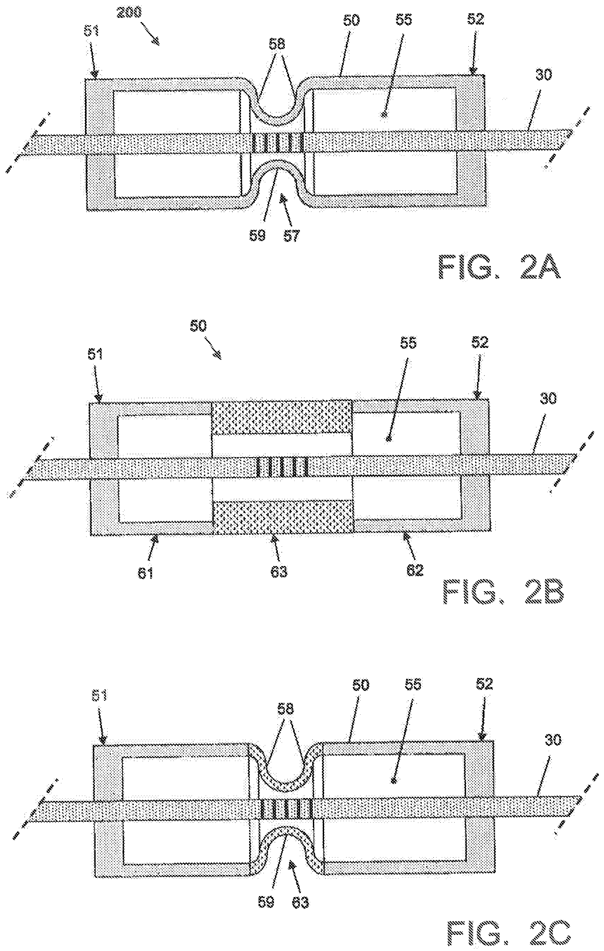

Figure 2A schematically shows a longitudinal cross section of a second embodiment of a sensing assembly 200 with increased pressure sensitivity according to the present invention. In this illustrated embodiment, the sensing body 50 is cylinder-shaped and provided with a circumferential groove 57. The groove 57 is preferably located halfway between the ends 51, 52. The groove 57 preferably has a U-shaped contour with a semi-circular bottom portion 58 and mutually parallel radial wall portions 59. The groove can be made of the same material as the remainder of the body 50, as shown.

Figure 2B illustrates an alternative embodiment, where the sensing body 50 comprises two body parts 61, 62 of a first material having a first axial stiffness, coupled together by a coupling piece 63 having a second axial stiffness less that the first axial stiffness. The precise dimensions of this coupling piece 63 are not critical, but the larger the axial length the larger is the effect obtained. Further, it is preferred that the outer diameter of the coupling piece 63 is equal to the outer diameter of the two body parts 61, 62, so that the outer cylinder surfaces are flush.

The enhancement in sensitivity is even greater if the coupling piece 63 is groove-shaped, as shown in figure 2C.

It is to be noted that the details of figure 1B can be combined with the details of any of figures 2A, 2B, 2C.

In stead of reducing the stiffness of the sensing body 50, or in addition thereto, measuring sensitivity of the sensing assembly can also be increased by adapting the stiffness of the fiber 30. As was explained in the above, any expansion or contraction of the sensing cylinder 50 is translated to a corresponding expansion or contraction of the fiber 30. This length variation is typically distributed evenly of the length of the fiber portion between the ends 51, 52 of the cylinder 50. However, the length of the FBG is smaller than the length of the fiber portion between the ends 51, 52 of the cylinder 50. Consequently, only a relatively small portion of the length variation of the sensing cylinder 50 is ultimately transferred to the FBG and translated into a measuring signal.

The present invention also provides a method for improving the sensitivity of an FBG sensor by changing the fiber design such that length variations are primarily concentrated at the location of the FBG. According to this inventive method, a fiber portion with an FBG is made with less stiffness that a fiber portion without any FBG. Because the fiber 30 is relatively stiff outside the FBG, any length variation of the sensing cylinder 50 is now concentrated to the FBG.

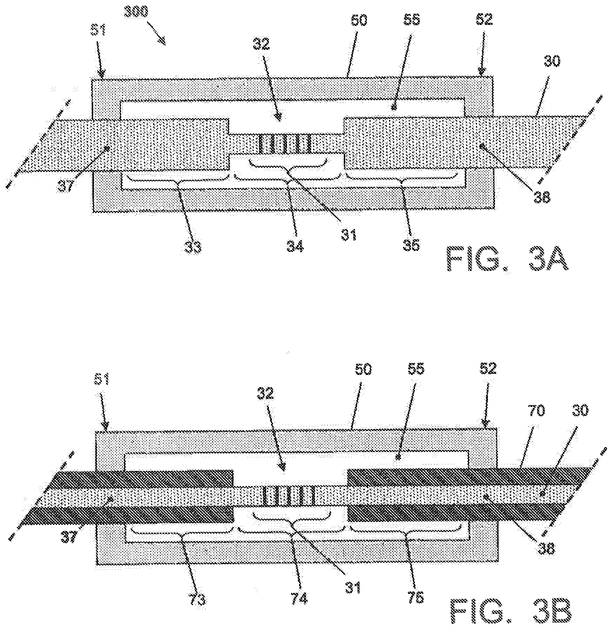

Figure 3A schematically shows a longitudinal cross section of a third embodiment of a sensing assembly 300 with increased sensitivity according to the present invention. The figure shows that the fiber 30 has a first thickness at the location of the attachment to the first and second ends 51, 52 of the sensing body 50, and that the fiber 30 has fiber portions 33, 35 having the first thinkness extending from these attachment locations it the hollow inner body space 55 towards the location of the FBG 32. The figure further shows that the fiber 30 has a central portion 34 of second thickness where the FBG 32 is located, the second thickness being less than the first thickness.

It is possible to manufacture this embodiment by starting with an FBG-free fiber having the first thickness, removing some of the fiber material at a certain fiber portion 34 to reduce the fiber thickness, and to then arrange an FBG at that fiber portion 34 with reduced thickness. It is alternatively possible to manufacture this embodiment by starting with a fiber 30 containing an FBG 32 in a certain fiber portion 34, and then removing some of the fiber material at said fiber portion 34 to reduce the fiber thickness while maintaining FBG functionality. In either case, the sensing body 50 is attached to the adapted fiber 30 later.

It is noted that the figure shows a steep step from first thickness to second thickness, but the transition may be more gradually than shown.

Figure 3B illustrates an alternative approach, where the fiber 30 is partially provided with an additional cladding 70 that is stiffer than the basic fiber 30 material. The additional cladding 70 may cover the fiber 30 outside the cylinder 50, as shown, but that is not necessary. The additional cladding 70 in any case covers the fiber 30 at the location of the ends 51, 52 of the cylinder 50 and extends from these ends to the location of the FBG 32, but does not cover the FBG 32. Particularly, the FBG 32 is located in a relatively thin fiber portion 74 that is free from said additional cladding (or where the additional cladding is thinner), which is sandwiched between fiber portions provided with respective cladding portions 73 and 75. As a result, any length variation of the sensing cylinder 50 is now concentrated to the length of the relatively thin fiber portion 74.

This aspect of the invention may be implemented by starting with any FBG-fiber 30 and add additional cladding portions 73, 75 outside the FBG 32, and then attach the sensing body 50.

Stiffening can also be achieved in other ways, for example by mounting or bonding the section of fiber that needs to be stiff on a stiff material. For example, over that section of fiber, it could be glued to a stiff plastic or metal material.

It is to be noted that the details of figure 3A or 3B can be combined with the details of any of figures 1A, 1B, 2A, 2B, 2C.

It is further noted that in the embodiments referring to Figures 1A, 1B, 2A, 2B, 2C, 3A and 3B, the hollow inner space may be filled with a compressible or incompressible filling material. Such filling material can be, e.g. a gel or a liquid selected depending on the particular application.

When a sensed physical parameter caused the length of the sensing body 50 to reduce, for instance an increased ambient pressure, the sensing portion 31 with the FBG is axially compressed. Without protective measures, the fiber 30 can not withstand axial compression and the fiber will consequently buckle, causing loss of measuring signal.

One possible way to overcome this problem is to apply bias tension on the fiber during manufacture. This will mean that, under atmospheric conditions, the fiber will be in a stressed state, and with increasing outside pressure the tension in the fiber will reduce.

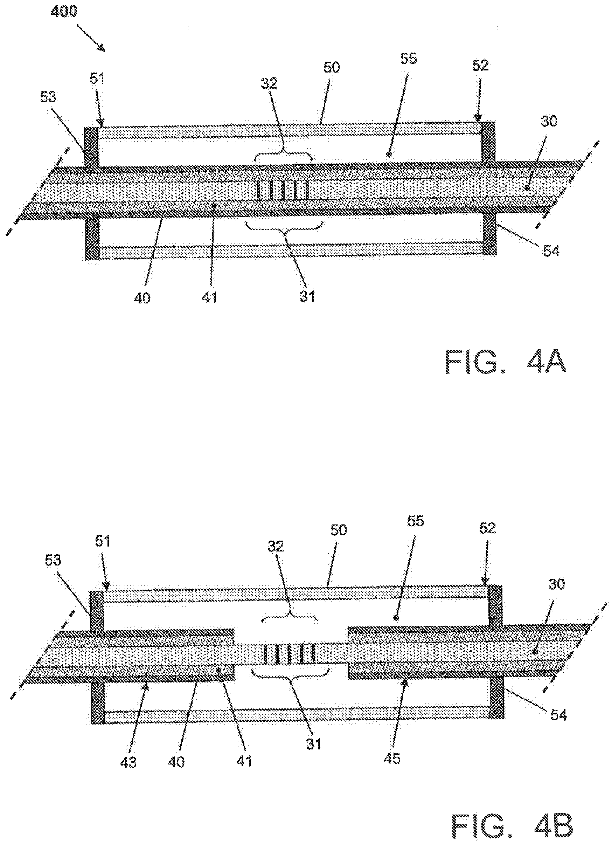

In a further elaboration of the present invention, an alternative solution is proposed. In this alternative solution, the fiber is radially supported by a capillary tube having higher stiffness than the fiber. This alternative solution is illustrated in figure 4.

Figure 4A schematically shows a longitudinal cross section of a fourth embodiment of a sensing assembly 400 according to the present invention. In this embodiment, the fiber 30 is arranged in a very narrow tube 40. This tube 40 has an inner diameter that is just slightly larger than the outer diameter of the fiber 30, giving sufficient play to pull the fiber 30 into the tube 40. By way of non-limiting example, for a (coated) fiber having an outer diameter of 150 µm, a suitable inner diameter for the tube 40 is 0.3 mm. In suitable embodiments, the difference between inner diameter of the tube 40 and outer diameter of the fiber 30 is within the range from 0.02 to 1 mm. This tube 40 will hereinafter be indicated as a capillary. As will be explained later in more detail, the capillary 40 is filled with a glue to ensure a good strain coupling between capillary 40 and fiber 30. The opposite ends 51, 52 of the cylinder 50 are attached to the capillary 40. Thus, attachment points of the fiber are attached to the body ends 51, 52 of the sensing body 50 via the capillary 40. In a preferred embodiment, the capillary 40 and the cylinder 50 are made from a metal, and the capillary 40 and the cylinder 50 are welded together. A material preferred for its weldability, resistability and durability is inconel.

The capillary 40 may extend over the entire length of the fiber 30, as shown in the figure. Inside the sensing body 50, the capillary 40 serves to radially support the fiber 30 to prevent it from buckling. Outside the sensing body 50, the capillary 40 serves as a protection of the fiber 30 against the environment, which is especially useful in embodiments that are intended for application in circumstances where the conditions are hostile and the fiber 30 needs to be protected, or in circumstances where the fiber should not contact the fluid medium for sanitary reasons, such as for instance medical applications, particularly intra-vascular applications. For instance in the case of steam-assisted oil extraction, the environment includes high temperature and high pressure steam and oil, often with high concentration presence of potentially harmful hydrogen.

The capillary 40 is made from a material that is resistent to the environment; a suitable material is for instance (stainless) steel and alloys. The optical fiber can also be already pre-coated with protective layers, like polyimide and carbon coatings.

For allowing the fiber 30 to be introduced into curved channels, the capillary 40 should have sufficient flexibility as regards bending, which means that the wall thickness of the capillary 40 should be sufficiently low. On the other hand, the wall thickness of the capillary 40 should be sufficiently high to provide sufficient structural robustness and integrity for allowing the assembly 400 to be pushed into narrow channels. A suitable wall thickness is for instance in the range 0.02-0.2 mm.

It is to be noted that in cases where protection of the fiber and/or contact between fiber and surroundings are not a factor of consideration, the capillary 40 may be omitted outside the sensing body 50.

The radial distance between the cylinder 50 and capillary 40 can be closed by the welding process. In a possible embodiment, the cylinder 50 has narrowed end portions matching the outer diameter of the capillary 40. In the embodiment as illustrated in figure 4, annular attachment members 53, 54 are arranged in between the capillary 40 and the cylinder 50, which are firmly attached to the capillary 40 and to the cylinder 50, for instance by welding.

It will thus be seen that an annular space 55 exists, confined in radial direction between the capillary 40 and the cylinder 50, and confined in axial direction between the opposite ends 51, 52 of the cylinder 50 or the annular attachment members 53, 54, respectively. This annular space 55 is gas-filled. The gas may for instance be air, or an inert gas filling such as nitrogen. This annular space 55 is aligned with the sensing fiber portion 31, or in other words the opposite ends 51, 52 of the cylinder 50 engage the capillary 40 at axially opposite sides of the sensing fiber portion 31.

The cylinder 50 is designed to have higher stiffness than the capillary 40. In an exemplary embodiment, the cylinder 50 is made from inconel, has an outer diameter of between 1.5 and 5 mm a wall thickness of 0.02 - 0.2 mm.

The operation is as follows. Under reference conditions, for instance 20°C and 1 bar pressure, the fiber 30 has a reference length and the cylinder 50 has a reference length. With increasing pressure, the cylinder 50 will be compressed in radial direction and in axial direction. The radial compression will influence the fiber 30 hardly or not. The axial compression is transferred to the capillary 40 and to the fiber 30, for which purpose the fiber 30 is bonded to the capillary 40, preferably over its entire length but in any case over the entire length of the cylinder 50. The bonding is such as to effect a good force transmission from the capillary 40 to the fiber 30 in axial direction. In an example, the bonding is effected by a suitable glue 41 arranged between the capillary 40 and the fiber 30.

In a preferred manufacturing process, the glue 41 is arranged in the capillary 40 and then the fiber 30 is introduced. In another preferred manufacturing process, the glue 41 is arranged on the fiber 30 and then the fiber is introduced into the capillary 40. In another preferred manufacturing process, the fiber is introduced into capillary 40, for instance pulled, and the glue 41 is supplied simultaneously at the entrance of the capillary 40 as the fiber 30 enters.

Thus, with increasing pressure, the fiber 30 will be subjected to increasing axial compression, resulting in reducing grating constant of the FBG or FBGs, which can be measured optically, as explained earlier. This applies to static pressure conditions but also to dynamic pressure variations. Due to the low inertia property of the design presented here, the resonance frequency and thus a large operation frequency bandwidth of the dynamic pressure sensor can be achieved.

It will thus be clear that the invention has succeeded in providing a design for a sensing assembly that combines sensitivity and durability with a very narrow cross section.

It is to be noted that the details of figure 4A can be combined with the details of any of figures 1A, 1B, 2A, 2B, 2C, 3A or 3B. Particularly, the glue-filled capillary 40 can perform as additional cladding 70 of figure 3B. Figure 4B illustrates an example of an embodiment where the capillary 40 is removed or omitted at the location of the FBG 32 to enhance the sensitivity of the FBG as described in the context of figure 3A and 3B. Capillary portions 43, 45 extend around fiber portions at opposite sides of the FBG 32, and are glued to these fiber portions.

It is further noted that in the embodiments as described referring to Figure 4A and Figure 4B the annular space 55 can be filled with filling material that is either compressible or incompressible.

It is specifically noted that in the embodiment shown in Fig. 1A, the annular space 55 can be filled with incompressible filling material. Such filling material can be a material whose compressibility is small enough to be neglected in relation to the changes during sensing action. In an embodiment, the incompressible filling material in the void can be a liquid or gel type.

In the particular application wherein the annular space 55 is filled with an incompressible filling material, as pressure increases, a net resulting force acting in a radial direction generates an expansion of the optical fiber 30 if the attachment members 53, 54 are not under a pressure that significantly counteracts the lengthening action due to radial load. Additionally, an isolating mechanism can be put in place so that an axial pressure force Fₐₓ does not counteract such expansion action on the fiber.

In one embodiment, a dampening mechanism as known in the art, such as a pair of bellows, can be used as an isolation mechanism isolating the expansion action exerted by the attachment members 53, 54 from axial forces due, e.g. to external pressure. In other embodiments, the bellow can be replaced by a rubber of a member with an air gap.

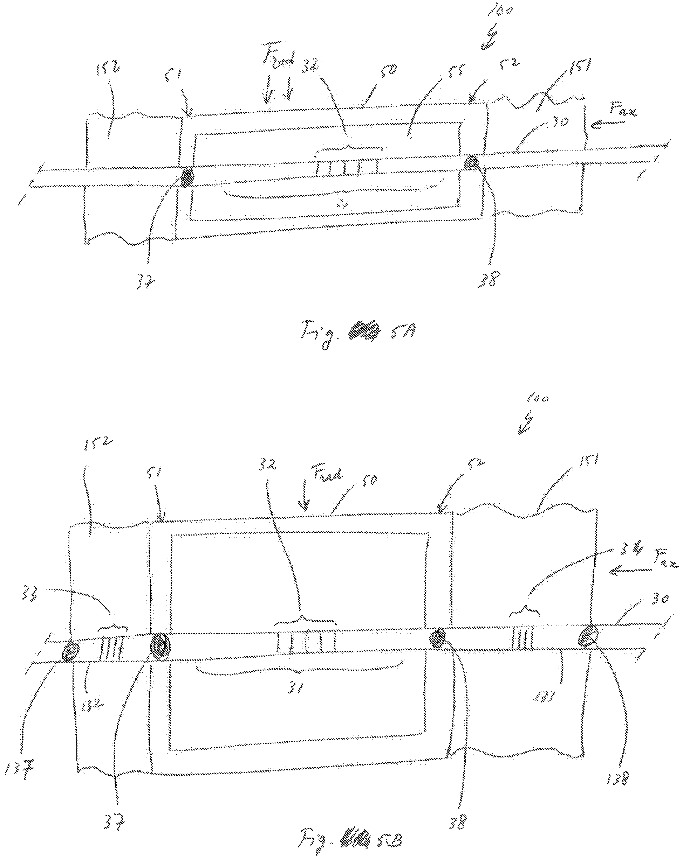

This embodiment has a further advantage in view of the embodiments of, e.g., figure 1A wherein the filling material is compressible. In the embodiment of figure 5A the fiber can be defined to be working in an elongation mode, that is, as pressure increases the length of the fiber also increases, on the other hand, on figure 1A the fiber works in a contraction mode, that is, the fiber compresses when there is an increased pressure. This difference has an important technical value because it means that on some embodiments of figure 1A the fiber has to be either pre-strained for the system to be able to measure or some kind of arrangement (such as a capillary tube) has to be put in place to prevent the fiber from bending. In the embodiment shown in figure 5A the assembly may operate without pre-straining the fiber and/or providing a capillary tube.

Figure 5A schematically shows a longitudinal cross section of a fifth embodiment of a sensing assembly 100. Again, the sensing body comprises a hollow space filled with an incompressible filling material. Compared to the embodiment shown in Fig. 1A, first and second pressure absorbing bodies implemented as bellows 151, 152, are provided as an isolating mechanism abutting the first and second body end 51, 52, respectively, of the sensing body 50. The first and second bellows 151, 152 are located at opposite sides of the sensing body 50 and preferably have a cylindrical shape having similar radial dimensions as the sensing body 50. Further, the bellows 151, 152 are located such that the optical fiber 30 traverses said bellows 151, 152. In a preferred embodiment, the fiber 30 is not rigidly connected in axial direction to the absorbing bodies 151, 152 such that the deformation of the absorbing bodies 151, 152 is largely unrestricted by the fiber 30, and the fiber 30 is not transmitting large axial forces to the attachment members 53, 54. Preferably, , an axial stiffness of the first and the second bellows 151, 152 is less than an axial stiffness of the sensing body 50. By designing the absorbing bodies such that their resulting axial stiffness is less than the axial stiffness of the sensing body, axial forces are at least partially absorbed. As an example, the bellows can be implemented as a mainly uniform body having the relatively small axial stiffness. However, alternative implementations are possible, e.g. applying absorbing bodies having elastic portions, e.g. having respective end faces, opposite to the sensing body 50, that are flexible, e.g. formed as membranes. Nonetheless, the bellows can also be replaced by isolating members being such members, independently of their stiffness capable of avoiding that axial forces affect the attachment members 53, 54.
In a preferred embodiment, the attachment members 53, 54 are highly flexible surfaces such as membranes that are connected to the sensing body 50 that may be an axially stiff and radially flexible body filled with low compressibility medium like gel and the bellows 151, 152 are axially flexible bodies such as bellows filled with high compressibility medium such as gases, like nitrogen or air. Sensing body 50 and attachment members 53 may be formed from the same material but can also be made from different bodies and assembled together. During operation, a pressure may be applied to the sensing assembly 100 resulting in radial forces, denoted by F_{rad}, and axial forces, denoted by Fₐₓ, exerted on the sensing assembly 100. While the axial forces may result in a length decrease, such inward movements of the attachment members are at least partially absorbed by the bellows 151, 152 such that the pressure effect on the external axial caps are not significantly transmitted to faces 51 and 52 while the radial forces on the sensing body 50 result in a decrease of diameter resulting in a push of the incompressible filling 55 axially to generate an outward deformation o flexible surfaces 51 and 52 that result in an elongation of the optical fiber 30 that can be measured by interrogating the Fiber Bragg Grating 31.

It is noted that, in principle, a sensing assembly 100 can be provided with a single pressure absorbing body, such as a bellow, abutting the first or second body end of the sensing body.

Figure 5B schematically shows a section comparable to Fig. 5A showing a variation of the embodiment of Fig. 5A. Here, a first and second portion 131, 132 of the optical fiber 30 traversing the pressure absorbing bodies implemented as bellows 131, 132 is attached, at a respective end section 137, 138 of said respective fiber portion 131, 132, to said first and second pressure absorbing body 137, 138, respectively. Further, said optical fiber sections 131, 132 include a further Fiber Bragg Grating 33, 34, so that any deformation such as a compression of the fiber in the pressure absorbing bodies 131, 132 can be measured. Alternatively, only a single optical fiber portion, traversing either the first or the second pressure absorbing body, can be provided with an additional Fiber Bragg Grating.

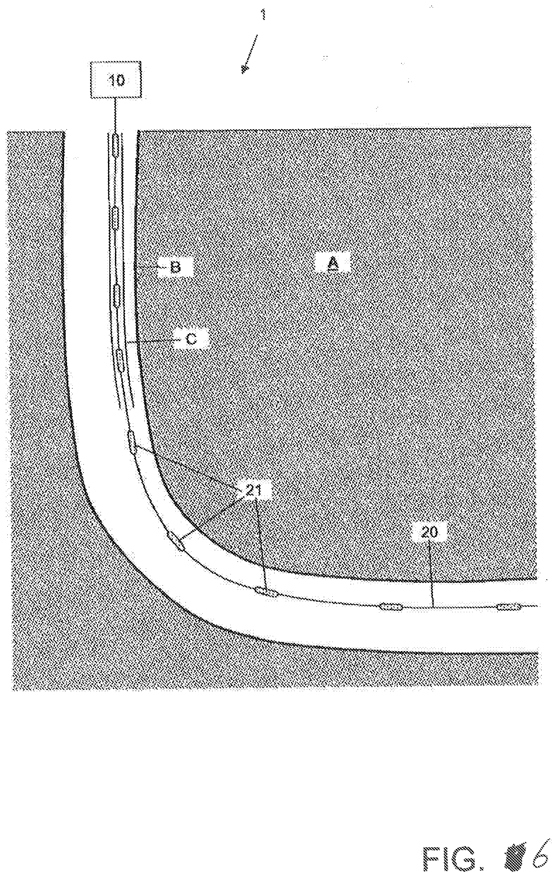

For illustrating a possible application of the invention, figure 6 schematically shows an oil well bore B arranged in Earth A. For sensing purposes, a narrow channel C is arranged within the bore B. The channel C typically has an internal diameter of about 8 mm, although the precise diameter of the channel is not critical, apart from the fact that sensing equipment should be narrow enough to fit within the channel. It is noted that the bore B and the channel C are just possible locations where the invention is to be applied; they do not form part of the invention itself.

Figure 6 also shows a pressure sensing system 1 according to the present invention, which comprises an elongate sensing assembly 20 arranged within the channel C. At different positions along the length of the sensing assembly 20, the sensing assembly 20 has a plurality of sensing areas 21.

By way of example, the pressure sensing system 1 shown in figure 6 comprises an interrogator device 10, that includes means for generating a light beam, means for receiving reflected light, and means for determining the wavelength (or spectrum) of the received light.

It is noted that in some embodiments the sensing assembly 20 only has one sensing area 21. In a possible embodiment, the elongate sensing assembly 20 may have a length of 100 m or more, for instance about 300 m. In a possible embodiment, the sensing areas 21 may have a mutual distance between 1 m and 100 m, for instance about 20 m. In a possible embodiment, the number of sensing areas 21 may be between 1 and 100, for instance 15.

The sensing assembly 20 may be implemented according to any of the assemblies 100, 200, 300, 400 discussed above. At each of the sensing areas 21, the sensing assembly 20 comprises at least one sensing body 50 associated to an individual or common fiber 30. The sensing assembly 20 may comprise a tubular guide for the fiber or fibers 30 for increased manoeuvrability.

It is noted that the use of the invention is not limited to the applications described above. By way of example, it is possible to use the same or adapted design for in-vivo blood pressure monitoring by sensing in arteries or capillaries. It will thus be clear that the invention has succeeded in providing a design for a sensing assembly that combines smooth outer shape with narrow profile which can facilitate its use in a body with minimal intrusion.

## Claims

1. Sensor assembly (100), comprising:
- an optical fiber (30) having a sensing fiber portion (31) that comprises a Fiber Bragg Grating (32);
- a sensing body (50) being size-responsive to the physical property being measured;
- wherein the sensing body (50) is filled with air or gas;
- the sensing body (50) having body ends (51, 52) attached to attachment points (37, 38) of the fiber (30) at opposite axial sides of the sensing fiber portion (31), **characterized in that** the sensing body (50) surrounds the sensing fiber portion (31), wherein the sensing body has a hollow inner space in which the fiber can move freely.

2. Sensor assembly according to claim 1, further comprising a capillary (40), wherein the optical fiber (30) is arranged in the capillary (40) and wherein the attachment points of the fiber are attached to the body ends (51, 52) of the sensing body (50) via the capillary (40).

3. Sensor assembly according to claim 2, wherein the optical fiber (30) is at least partially attached to the capillary (40).

4. Sensor assembly according to claim 2 or 3, wherein the capillary (40) extends at least over the entire length or over the entire length of the sensing body (50) except at the location of the FBG (32).

5. Sensor assembly according to claim 2, 3 or 4, wherein the capillary (40) has capillary portions (43, 45) extending over fiber portions adjacent the FBG (32) while the FBG (32) is free from capillary (40).

6. Sensor assembly according to claim 5, wherein a capillary portion extends from the body ends (51, 52) of the sensing body (50) to a location adjacent the FBG (32).

7. Sensor assembly according to any of the claims 2-6, wherein the capillary (40) extends outwardly beyond the sensing body (50) to protect the fiber (30).

8. Sensor assembly according to any of the claims 2-7, wherein the sensing body (50) has an outer diameter in the range of 1-5 mm.

9. Sensor assembly according to any of the claims 2-8 for in-vivo and/or intra-vascular blood pressure measurement, wherein at least the capillary (40) is made of a biocompatible material.

10. Sensor assembly according to any of the claims 1-9, wherein the sensing body comprises a hollow space filled with an incompressible filling material.

11. Sensor assembly according to claim 10, further comprising a first isolating mechanism abutting a first body end (51;52) of the sensing body (50) such that the optical fiber (30) traverses said first isolating mechanism.

12. Sensor assembly according to claim 11, further comprising a second isolating mechanism abutting a second body end (51; 52) of the sensing body (50), opposite to the first body end (51; 52) of the sensing body, such that the optical fiber (30) traverses said second isolating mechanism.

13. Sensor assembly according to claim 11 or 12, wherein the first and/or second isolating mechanism is a pressure absorbing body (151, 152).

14. Sensor assembly according to claim 13, wherein an axial stiffness of the pressure absorbing bodies (151, 152) is less than an axial stiffness of the sensing body (50).

15. Sensor assembly according to any of the preceding claims 11-14, wherein a portion (131, 132) of the optical fiber (30) traversing the first or second pressure absorbing body (151, 152) is attached, at an end section (137, 138) thereof, to said first or second pressure absorbing body (151, 152), and wherein said optical fiber section comprises a further Fiber Bragg Grating (32, 33).

16. Sensor assembly according to any of the preceding claims 3-15, wherein the capillary (40) is preferably filled with glue (41) for attaching the optical fiber (30) to the capillary (40).

## Patentansprüche

1. Sensorbaugruppe (100), umfassend:
- eine optische Faser (30) mit einem Erfassungsfaserabschnitt (31), der ein Faser-Bragg-Gitter (32) umfasst;
- einen Erfassungskörper (50), der auf die zu messende physikalische Eigenschaft größenabhängig reagiert;
- wobei der Erfassungskörper (50) mit Luft oder Gas gefüllt ist;
- der Erfassungskörper (50) Körperenden (51, 52) aufweist, die an Befestigungspunkten (37, 38) der Faser (30) an gegenüberliegenden axialen Seiten des Erfassungsfaserabschnitts (31) angebracht sind, **dadurch gekennzeichnet, dass** der Erfassungskörper (50) den Erfassungsfaserabschnitt (31) umgibt, wobei der Erfassungskörper einen hohlen Innenraum aufweist, in dem sich die Faser frei bewegen kann.

2. Sensorbaugruppe nach Anspruch 1, ferner umfassend eine Kapillare (40), wobei die optische Faser (30) in der Kapillare (40) angeordnet ist und wobei die Befestigungspunkte der Faser an den Körperenden (51, 52) des Erfassungskörpers (50) über die Kapillare (40) angebracht sind.

3. Sensorbaugruppe nach Anspruch 2, wobei die optische Faser (30) wenigstens teilweise an der Kapillare (40) angebracht ist.

4. Sensorbaugruppe nach Anspruch 2 oder 3, wobei sich die Kapillare (40) wenigstens über die gesamte Länge oder über die gesamte Länge des Erfassungskörpers (50) mit Ausnahme der Stelle des FBG (32) erstreckt.

5. Sensorbaugruppe nach Anspruch 2, 3 oder 4, wobei die Kapillare (40) Kapillarabschnitte (43, 45) aufweist, die sich über Faserabschnitte neben dem FBG (32) erstrecken, während das FBG (32) frei von Kapillare (40) ist.

6. Sensorbaugruppe nach Anspruch 5, wobei sich ein Kapillarabschnitt von den Körperenden (51, 52) des Erfassungskörpers (50) zu einer Stelle neben dem FBG (32) erstreckt.

7. Sensorbaugruppe nach einem der Ansprüche 2 bis 6, wobei sich die Kapillare (40) nach außen über den Erfassungskörper (50) hinaus erstreckt, um die Faser (30) zu schützen.

8. Sensorbaugruppe nach einem der Ansprüche 2 bis 7, wobei der Erfassungskörper (50) einen Außendurchmesser im Bereich von 1 bis 5 mm aufweist.

9. Sensorbaugruppe nach einem der Ansprüche 2 bis 8 zur in-vivo und/oder intra-vaskulären Blutdruckmessung, wobei wenigstens die Kapillare (40) aus einem biokompatiblen Material besteht.

10. Sensorbaugruppe nach einem der Ansprüche 1 bis 9, wobei der Erfassungskörper einen Hohlraum umfasst, der mit einem inkompressiblen Füllmaterial gefüllt ist.

11. Sensorbaugruppe nach Anspruch 10, ferner umfassend einen ersten Isoliermechanismus, der an ein erstes Körperende (51; 52) des Erfassungskörpers (50) grenzt, so dass die optische Faser (30) den ersten Isoliermechanismus durchquert.

12. Sensorbaugruppe nach Anspruch 11, ferner mit einem zweiten Isoliermechanismus, der an ein zweites Körperende (51; 52) des Erfassungskörpers (50) gegenüber dem ersten Körperende (51; 52) des Erfassungskörpers grenzt, so dass die optische Faser (30) den zweiten Isoliermechanismus durchquert.

13. Sensorbaugruppe nach Anspruch 11 oder 12, wobei der erste und/oder zweite Isoliermechanismus ein Druckabsorptionskörper (151, 152) ist.

14. Sensorbaugruppe nach Anspruch 13, wobei eine axiale Steifheit der Druckabsorptionskörper (151, 152) geringer ist als eine axiale Steifheit des Erfassungskörpers (50).

15. Sensorbaugruppe nach einem der vorhergehenden Ansprüche 11 bis 14, wobei ein Abschnitt (131, 132) der optischen Faser (30), die den ersten oder zweiten Druckabsorptionskörper (151, 152) durchquert, an einem Endabschnitt (137, 138) davon an den ersten oder den zweiten Druckabsorptionskörper (151, 152) angebracht ist und wobei der optische Faserabschnitt ein weiteres Faser-Bragg-Gitter (32, 33) umfasst.

16. Sensorbaugruppe nach einem der vorhergehenden Ansprüche 3 bis 15, wobei die Kapillare (40) vorzugsweise mit Klebstoff (41) gefüllt ist, um die optische Faser (30) an der Kapillare (40) anzubringen.

## Revendications

1. Ensemble capteur (100), comprenant :
- une fibre optique (30) ayant une portion fibre de détection (31) qui comprend un réseau de Bragg sur fibre (32) ;
- un corps de détection (50) étant d'une taille sensible à la propriété physique étant mesurée ;
- dans lequel le corps de détection (50) est rempli d'air ou de gaz ;
- le corps de détection (50) ayant des extrémités de corps (51, 52) attachées à des points d'attache (37, 38) de la fibre (30) au niveau de côtés axiaux opposés de la portion fibre de détection (31), **caractérisé en ce que** le corps de détection (50) entoure la portion fibre de détection (31), dans lequel le corps de détection a un espace intérieur creux dans lequel la fibre peut se déplacer librement.

2. Ensemble capteur selon la revendication 1, comprenant en outre un capillaire (40), dans lequel la fibre optique (30) est agencée dans le capillaire (40) et dans lequel les points d'attache de la fibre sont attachés aux extrémités de corps (51, 52) du corps de détection (50) via le capillaire (40).

3. Ensemble capteur selon la revendication 2, dans lequel la fibre optique (30) est au moins partiellement attachée au capillaire (40).

4. Ensemble capteur selon la revendication 2 ou 3, dans lequel le capillaire (40) s'étend au moins sur la longueur entière ou sur la longueur entière du corps de détection (50) à l'exception de l'emplacement du RBF (32).

5. Ensemble capteur selon la revendication 2, 3 ou 4, dans lequel le capillaire (40) a des portions de capillaire (43, 45) s'étendant sur des portions de fibre adjacentes au RBF (32) tandis que le RBF (32) est dépourvu de capillaire (40).

6. Ensemble capteur selon la revendication 5, dans lequel une portion de capillaire s'étend à partir des extrémités de corps (51, 52) du corps de détection (50) jusqu'à un emplacement adjacent au RBF (32).

7. Ensemble capteur selon l'une quelconque des revendications 2 à 6, dans lequel le capillaire (40) s'étend vers l'extérieur au-delà du corps de détection (50) pour protéger la fibre (30).

8. Ensemble capteur selon l'une quelconque des revendications 2 à 7, dans lequel le corps de détection (50) a un diamètre extérieur dans la plage de 1 à 5 mm.

9. Ensemble capteur selon l'une quelconque des revendications 2 à 8 pour une mesure de la pression sanguine *in vivo* et/ou intravasculaire, dans lequel au moins le capillaire (40) est fabriqué à partir d'un matériau biocompatible.

10. Ensemble capteur selon l'une quelconque des revendications 1 à 9, dans lequel le corps de détection comprend un espace creux rempli d'un matériau de remplissage incompressible.

11. Ensemble capteur selon la revendication 10, comprenant en outre un premier mécanisme isolant venant en butée contre une première extrémité de corps (51 ; 52) du corps de détection (50) de telle manière que la fibre optique (30) traverse ledit premier mécanisme isolant.

12. Ensemble capteur selon la revendication 11, comprenant en outre un second mécanisme isolant venant en butée contre une seconde extrémité de corps (51 ; 52) du corps de détection (50), opposée à la première extrémité de corps (51 ; 52) du corps de détection, de telle manière que la fibre optique (30) traverse ledit second mécanisme isolant.

13. Ensemble capteur selon la revendication 11 ou 12, dans lequel le premier et/ou second mécanisme isolant est un corps absorbant la pression (151, 152).

14. Ensemble capteur selon la revendication 13, dans lequel une rigidité axiale des corps absorbant la pression (151, 152) est inférieure à une rigidité axiale du corps de détection (50).

15. Ensemble capteur selon l'une quelconque des revendications 11 à 14 précédentes, dans lequel une portion (131, 132) de la fibre optique (30) traversant le premier ou le second corps absorbant la pression (151, 152) est attachée, au niveau d'une section d'extrémité (137, 138) de celui-ci, audit premier ou second corps absorbant la pression (151, 152), et dans lequel ladite section de fibre optique comprend un réseau de Bragg sur fibre (32, 33) supplémentaire.

16. Ensemble capteur selon l'une quelconque des revendications 3 à 15 précédentes, dans lequel le capillaire (40) est de préférence rempli de colle (41) pour attacher la fibre optique (30) au capillaire (40).
